(19) Europäisches Patentamt
European Patent Office
Office européen des brevets

(11) Veröffentlichungsnummer: **0 042 569**
**B1**

(12) # EUROPÄISCHE PATENTSCHRIFT

(45) Veröffentlichungstag der Patentschrift: **12.10.83**

(21) Anmeldenummer: **81104605.1**

(22) Anmeldetag: **15.06.81**

(51) Int. Cl.³: **C 07 D 495/10,** C 07 D 495/20, C 07 D 211/90 // (C07D495/10, 333/00, 221/00),(C07D495/20, 333/00, 221/00, 221/00)

(54) **4,4-Disubstituierte Spiro-1,4-dihydropyridine, ein Verfahren zu ihrer Herstellung, sowie ihre Verwendung als Zwischenprodukt.**

(30) Priorität: **25.06.80 DE 3023820**

(43) Veröffentlichungstag der Anmeldung: **30.12.81 Patentblatt 81/52**

(45) Bekanntmachung des Hinweises auf die Patenterteilung: **12.10.83 Patentblatt 83/41**

(84) Benannte Vertragsstaaten: **AT BE CH DE FR GB IT LI NL SE**

(56) Entgegenhaltungen: **DE-A-2 117 571**

(73) Patentinhaber: **BAYER AG, Zentralbereich Patente, Marken und Lizenzen, D-5090 Leverkusen 1, Bayerwerk (DE)**

(72) Erfinder: **Goldmann, Siegfried, Dr., Kuckuckstrasse 41, D-5600 Wuppertal 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

4,4-Disubstituierte Spiro-1,4-dihydropyridine, ein Verfahren zu ihrer Herstellung, sowie ihre Verwendung als Zwischenprodukt

Die vorliegende Erfindung betrifft ein neues, chemisch eigenartiges Verfahren zur Herstellung von neuen 4,4-disubstituierten Spiro-1,4-dihydropyridinen, die als Zwischenprodukte für die Synthese von 4,4-disubstituierten 1,4-Dihydropyridinen verwendet werden können, sowie neue Verbindungen aus dieser Stoffklasse.

In 4-Position monosubstituierte 1,4-Dihydropyridine sind durch «Hantzsch-Synthese» leicht zugänglich und bereits bekannt (vgl. A. Hantzsch Justus Lieb. Ann. Chem. 215, 1 (1882) und DT-OS 2 117 571).

1,4-Dihydropyridin-3,5-dicarbonsäureesterderivate, die in 4-Position zwei Substituenten tragen, sind nach bekannten Methoden bisher nicht herstellbar (vgl. B. Loev und K. M. Snader, J. Org. Chem. 30, 1914 (1965)). Auch die Addition von metallorganischen Verbindungen an Pyridinderivaten lässt sich nur durchführen, wenn die 4-Position des Pyridins nicht bereits substituiert ist (vgl. R. Lukes und J. Kuthan, Collect. Czech. Chem. Comm. 26, 1845 (1961); J. Palacek, K. Vondra und J. Kuthan, ibid. 34, 2991 (1969) und J. F. Biellmann, H. J. Callot und M. P. Goeldner, Tetrahedron 26, 4655 (1970).

Die vorliegende Erfindung betrifft neue 4,4-disubstituierte Spiro-1,4-dihydropyridine der allgemeinen Formel (I)

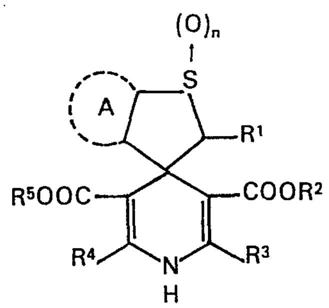

(I)

in welcher

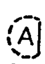 für Aryl, Furyl, Pyrrolyl, Pyrazolyl, Imidazolyl, Oxazolyl, Isoxazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei der Arylrest sowie die genannten Heterocyclen gegebenenfalls 1, 2 oder 3 gleiche oder verschiedene Substituenten aus der Gruppe Phenyl, Alkyl, Alkenyl, Alkinyl, Alkoxy, Alkylen, Dioxialkylen, Halogen, Trifluormethyl, Trifluormethoxy, Alkylamino, Nitro, Cyano, Azido oder Carbonamido enthalten,

$R^3$ und $R^4$ gleich oder verschieden sind und jeweils für Wasserstoff oder einen geradkettigen oder verzweigten Alkylrest ohne asymmetrisches Kohlenstoffatom, einen Arylrest oder einen Aralkylrest stehen,

$R^2$ und $R^5$ gleich oder verschieden sind und jeweils für einen geradkettigen oder verzweigten aliphatischen oder cycloaliphatischen, gesättigten oder ungesättigten Kohlenwasserstoffrest stehen, der durch ein Sauerstoffatom in der Kette unterbrochen sein kann, und der gegebenenfalls substituiert ist durch Halogen, Pyridyl, Phenyl oder Phenoxy, wobei die Phenylgruppen ihrerseits substituiert sein können durch Halogen, Cyano, Dialkylamino, Alkoxy, Alkyl, Trifluormethyl oder Nitro,

$R^1$ für Wasserstoff, einen geradkettigen oder verzweigten aliphatischen oder cycloaliphatischen, gesättigten oder ungesättigten Kohlenwasserstoffrest steht, der gegebenenfalls durch ein Sauerstoffatom in der Kette unterbrochen ist und der gegebenenfalls substituiert ist durch Phenyl, Phenoxy, Pyridyl oder Amino, wobei der Phenyl- und der Phenoxyrest ihrerseits gegebenenfalls substituiert sind durch Halogen, Cyano, Dialkylamino, Alkoxy, Alkyl, Trifluormethyl oder Nitro und wobei die Aminogruppe gegebenenfalls substituiert ist durch zwei gleiche oder verschiedene Substituenten aus der Gruppe Alkyl, Alkoxyalkyl, Aryl oder Aralkyl, und

n für 0, 1 oder 2 steht.

Die Erfindung betrifft weiterhin ein Verfahren zur Herstellung von Verbindungen der allgemeinen Formel (I), dadurch gekennzeichnet, das man Pyridine der allgemeinen Formel (II)

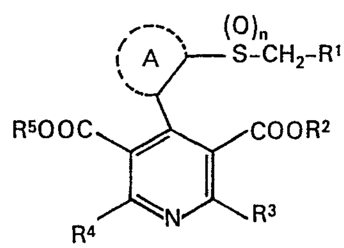

(II)

in welcher

$R^1$, $R^2$, $R^3$, $R^4$, $R^5$, n und A die oben angegebene Bedeutung haben,

bei Temperaturen zwischen −120°C bis +30°C in Gegenwart von mindestens äquivalenten Mengen an Metallhydriden, Metallamiden oder Metallalkoholaten und gegebenenfalls in Gegenwart von inerten organischen Lösungsmitteln zu Spiroverbindungen der allgemeinen Formel (I) cyclisiert.

Verwendet man 2,6-Dimethyl-4-(2-methylsulfinyl-phenyl)-pyridin-3,5-dicarbonsäure-dimethyl-ester als Ausgangsstoff, so kann der Reaktionsablauf durch das folgende Formelschema wiedergegeben werden.

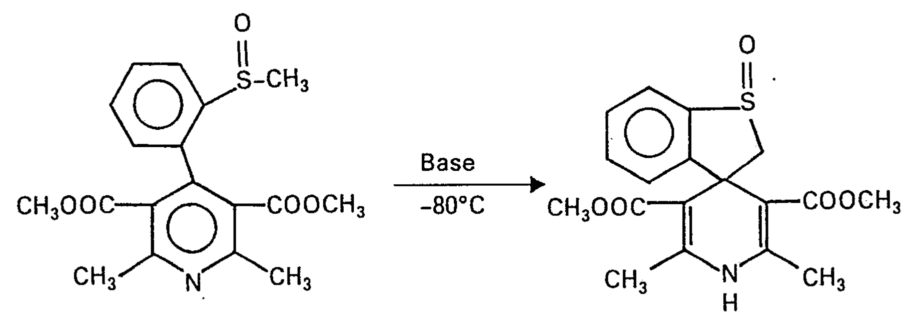


Die als Ausgangsstoffe verwendeten Pyridinverbindungen der allgemeinen Formel (II) sind bisher noch nicht bekannt, sind jedoch nach bekannten Oxidationsverfahren wie z.B. Oxidation mit Chloranil (vgl. J. Am. Chem. Soc. 79, 3477 (1957)) aus den entsprechenden 1,4-Dihydropyridinen hergestellt worden. Die Herstellung der 1,4-Dihydropyridine erfolgt ebenfalls nach bekannten Methoden (vgl. DT-OS 2 117 571; J. Med. Chem. 17, 956 (1974)).

Als inerte organische Lösungsmittel seien vorzugsweise genannt Ether, wie z.B. Diethylether, Tetrahydrofuran oder Dioxan, Kohlenwasserstoffe wie z.B. Benzin, Petrolether, Toluol oder Xylol.

Als erfindungsgemäss einsetzbare Metallhydride, -amide und -alkoholate seien vorzugsweise genannt Natriumhydrid, Kaliumhydrid, Metalldiethylamid oder -diisopropylamid, Kalium-t-butylat oder Natriumethylat.

Bei der Durchführung des erfindunsgemässen Verfahrens arbeitet man vorzugsweise in einem Temperaturbereich von −100°C bis +20°C, insbesondere in einem Bereich zwischen −90°C bis −40°C.

Die Umsetzung erfolgt üblicherweise bei Normaldruck, kann aber auch bei erhöhtem Druck durchgeführt werden. Die zum Ringschluss eingesetzte Base wird vorzugsweise mindestens in äquivalenten Mengen eingesetzt. Ein 2- bis 3-facher Überschuss an Base kann gegebenenfalls vorteilhaft sein.

Bei Kenntnis des Standes der Technik konnte nicht erwartet werden, dass die Verbindungen der Formel (II) unter den angegebenen Verfahrensbedingungen zu 4,4-disubstituierten 1,4-Dihydropyridinen cyclisieren, zumal aus dem Stande der Technik bekannt war, dass eine Zweitsubstitution der 4-Position durch Addition von metallorganischen Verbindungen an Pyridinderivate nicht möglich ist. Es ist daher als ausgesprochen überraschend zu bezeichnen, das diese neuen und bisher nicht zugänglichen Verbindungen durch ein so einfaches Verfahren hergestellt werden können.

Von besonderem Interesse sind Verbindungen der allgemeinen Formel (I), in welcher

(A) für Phenyl, Naphthyl, Furyl, Pyrrolyl oder Pyridyl steht, wobei diese Ringe gegebenenfalls 1, 2 oder 3 gleiche oder verschiedene Substituenten aus der Gruppe Phenyl, Alkyl, Alkenyl, Alkinyl, Alkoxy, Alkylen, Dioxyalkylen, Alkylamino mit jeweils bis zu 4 Kohlenstoffatomen, Halogen, Trifluormethyl, Trifluormethoxy, Nitro, Cyano, Acido oder Carbonamido enthalten,

$R^3$ und $R^4$ gleich oder verschieden sind und jeweils für Wasserstoff oder einen Alkylrest ohne asymmetrisches Kohlenstoffatom mit bis zu 4 Kohlenstoffatomen, einen Phenylrest oder einen Benzylrest stehen,

$R^2$ und $R^5$ gleich oder verschieden sind und jeweils für einen geradkettigen oder verzweigten aliphatischen oder cycloaliphatischen gesättigten oder ungesättigten Kohlenwasserstoffrest mit bis zu 6 Kohlenstoffatomen stehen, der durch ein Sauerstoffatom in der Kette unterbrochen sein kann und der gegebenenfalls substituiert ist durch Halogen, Pyridyl, Phenyl oder Phenoxy, wobei die Phenylgruppen ihrerseits substituiert sein können durch Halogen, Cyano, Nitro oder Trifluormethyl,

$R^1$ für Wasserstoff, einen geradkettigen oder verzweigten aliphatischen oder cycloaliphatischen Kohlenwasserstoffrest mit bis zu 8 Kohlenstoffatomen steht, der gegebenenfalls in der Kette durch ein Sauerstoffatom unterbrochen ist und der gegebenenfalls substituiert ist durch Phenyl, Phenoxy, Pyridyl oder Amino, wobei der Phenyl- und der Phenoxyrest gegebenenfalls substituiert sind durch Halogen, Cyano, Trifluormethyl, Nitro, Alkyl, Alkoxy, oder Dialkylamino mit jeweils 1 bis 4 Kohlenstoffatomen, und wobei die Aminogruppe gegebenenfalls substituiert ist durch zwei gleiche oder verschiedene Substituenten aus der Gruppe Alkyl, Alkoxyalkyl mit jeweils bis zu 4 Kohlenstoffatomen, Phenyl oder Benzyl, und

n für 0, 1 oder 2 steht.

Vorzugsweise seien solche Verbindungen der allgemeinen Formel (I) genannt, in welcher A für Phenyl, Furyl, Pyrrolyl oder Pyridyl steht, wobei die genannten Ringe gegebenenfalls 1 oder 2 gleiche oder verschiedene Substituenten aus der Gruppe Phenyl, Alkyl, Alkoxy, Alkylamino, mit jeweils 1 bis 4 Kohlenstoffatomen in den Alkyl- und Alkoxy- resten, Halogen, Trifluormethyl, Trifluormethoxy, Nitro, Cyano oder Acido enthalten,

$R^3$ und $R^4$ gleich oder verschieden sind und jeweils für Wasserstoff, Alkyl ohne asymmetrisches Kohlenstoffatom mit 1 bis 4 Kohlenstoffatomen, Phenyl oder Benzyl stehen,

$R^2$ und $R^5$ gleich oder verschieden sind und jeweils für einen Alkylrest mit 1 bis 6 Kohlenstoffatomen stehen, der gegebenenfalls durch ein Sauerstoffatom in der Kette unterbrochen ist

oder der gegebenenfalls durch Halogen oder Phenyl substituiert ist,
$R^1$ für Wasserstoff, Alkyl oder Alkinyl mit bis zu 6 Kohlenstoffatomen steht, wobei der Alkylrest gegebenenfalls durch ein Sauerstoffatom in der Kette unterbrochen ist und gegebenenfalls substituiert ist durch Phenyl, Phenoxy oder Amino, wobei die Aminogruppe gegebenenfalls substituiert ist durch zwei gleiche oder verschiedene Substituenten aus der Gruppe Alkyl mit 1 bis 4 Kohlenstoffatomen, Phenyl oder Benzyl und
n für 0 oder 1 steht.

Die erfindungsgemässen Verbindungen der Formel (I) lassen sich auf einfache Weise nach bekannten Methoden z.B. durch Behandlung mit Raney-Nickel in schwefelfreie Produkte der allgemeinen Formel (III)

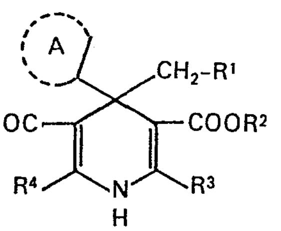

(III)

überführen (vgl. Org. React. 12, 356–529 (1962) und Chem. Rev. 62, 374–404 (1962). Diese 4,4-disubstituierten 1,4-Dihydropyridine der allgemeinen Formel (III) stellen wertvolle pharmazeutisch wirksame Verbindungen dar, die zur Behandlung von Kreislauferkrankungen eingesetzt werden können.

Das erfindungsgemässe Verfahren und die so hergestellten neuen Verbindungen stellen somit einen vorteilhaften Weg dar zur Herstellung von neuen pharmazeutisch wirksamen Stoffen.

Verbindungen der allgemeinen Formel (I), in welcher n 0 bedeutet, können in vorteilhafter Weise auch dadurch erhalten werden, dass man die entsprechenden Sulfinylverbindungen mittels Raney-Nickel zu den Benzothiophenverbindungen reduziert (vgl. Beispiele 7, 9).

Die nachfolgenden Beispiele sollen das erfindungsgemässe Verfahren erläutern ohne es einzuschränken.

Beispiel 1

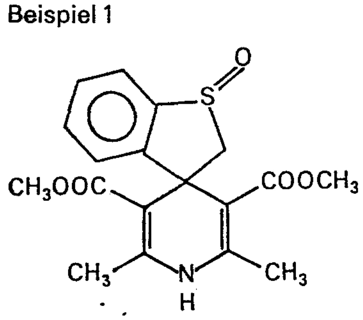

150 mmol 2,6-Dimethyl-4-(2-methylsulfinyl-phenyl)-pyridin-3,5-dicarbonsäure-dimethylester werden in 500 ml wasserfreiem Tetrahydrofuran gelöst und bei −78°C mit 300 mmol Lithiumdiisopropylamid versetzt. Unmittelbar darauf werden 50 ml Methanol, festes Ammoniumchlorid und 1 l Wasser zugesetzt. Der Niederschlag wird abgesaugt, in Wasser gewaschen und bei 100°C getrocknet. Man erhält 4,3′-Spiro [2,6-dimethyl-3,5-bismethoxycarbonyl-1,4-dihydropyridin]-[2′,3′-dihydro-1′-benzothiophen-1′-oxid] vom Fp.: 286–289°C (Zers.).

Ausbeute: 80% der Theorie.

Beispiel 2

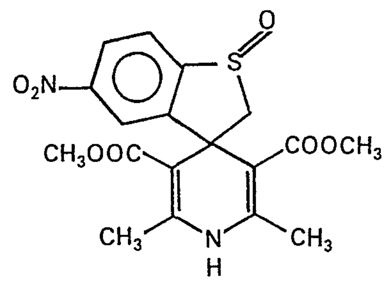

135 mmol 2,6-Dimethyl-4-(2-methylsulfinyl-5-nitrophenyl)-pyridin-3,5-dicarbonsäuredimethylester werden in Diethylether gelöst und bei 20°C mit 270 mmol Kaliumhydrid umgesetzt und protoniert. Das Lösungsmittel wird abrotiert, in Mehylenchlorid aufgenommen, getrocknet und erneut einrotiert. Der Rückstand kristallisiert beim Verreiben mit Essigester.

Man erhält 4,3′-Spiro [2,6-dimethyl-3,5-bismethoxy-carbonyl-1,4-dihydropyridin]-[5′-nitro-2′,3′-dihydro-1′-benzothiophen-1′-oxid] vom Fp.: 257–259°C (Zers.).

Ausbeute: 33% der Theorie.

Beispiel 3

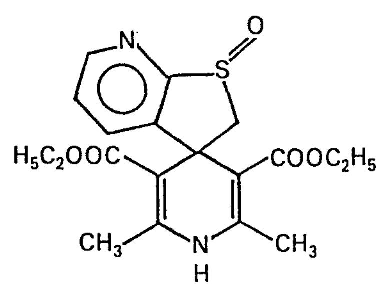

21 mmol 2,3-Dimethyl-4-(2-methylsulfinylpyridyl-3)-pyridin-3,5-dicarbonsäurediethylester werden in Dioxan gelöst und analog Beispiel 1 mit Lithiumdiethylamid umgesetzt und aufgearbeitet.

Man erhält 4,3′-Spiro [2,6-dimethyl-3,5-bismethoxy-carbonyl-1,4-dihydropyridin]-[thieno-[2,3,6]pyridin-2′,3′-dihydro-1′-oxid] vom Fp.: 253–256°C.

Ausbeute: 27% der Theorie.

Beispiel 4

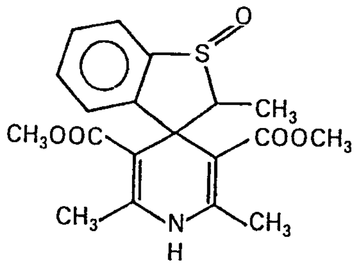

125 mmol 2,6-Dimethyl-4-(2-ethylsulfinylphenyl)-pyridin-3,5-dicarbonsäuredimethylester werden analog Beispiel 1 mit Lithiumdiisopropylamid bei −100°C umgesetzt und protoniert. Das Lösungsmittel wird abrotiert, der Rückstand mit Methylenchlorid extrahiert, über $Na_2SO_4$ getrocknet und einrotiert. Der Rückstand wird in Aceton gelöst und mit Ether gefällt. Man erhält 4,4'-Spiro [2,6-dimethyl-3,5-bismethoxycarbonyl-1,4-dihydro-pyridin]-[2'-methyl-2',3'-dihydro-1'-benzothiophen-1'-oxid] als Diastereomerengemisch mit dem Fp.: 200–206°C.

Ausbeute: 41% der Theorie.

Beispiel 5

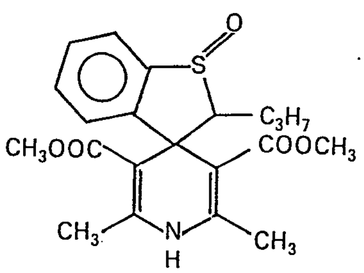

61 mmol 2,6-Dimethyl-4-(2-propylsulfinylphenyl)-pyridin-3,5-dicarbonsäuredimethylester werden in Toluol gelöst und analog Beispiel 1 umgesetzt. Es verbleiben 4,3'-Spiro [2,6-dimethyl-3,5-bismethoxycarbonyl-1,4-dihydropyridin]-[2'-propyl-2',3'-dihydro-1'-benzothiophen-1'-oxid] vom Fp.: 225–229°C.

Ausbeute: 52% der Theorie.

Beispiel 6

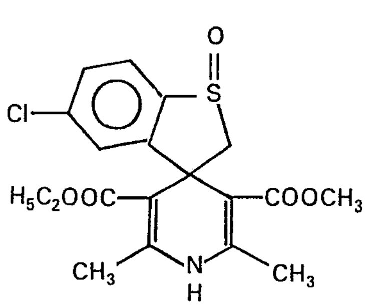

50 mmol 2,6-Dimethyl-4-(2-methylsulfinyl-5-chlor-phenyl)-pyridin-3,5-dicarbonsäure-methylethylester werden analog Beispiel 1 umgesetzt. Man erhält 4,3'-Spiro [2,6-dimethyl-3-methoxycarbonyl-5-ethoxycarbonyl-1,4-dihydropyridin]-[5'-chlor-2',3'-dihydro-1'-benzothiophen-1'-oxid] vom Fp.: 210–215°C.

Ausbeute: 44% der Theorie.

Beispiel 7

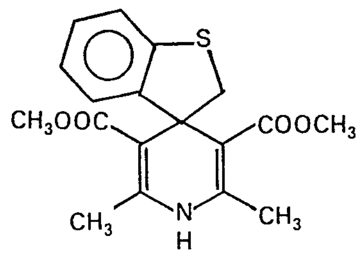

28 mmol 4,3'-Spiro [2,6-dimethyl-3,5-bismethoxy-carbonyl-1,4-dihydropyridin]-[2',3'-dihydro-1'-benzothiophen-1'-oxid] (Beispiel 1) werden in 500 ml Aceton gelöst und mit 100 g Raney-Nickel (in $H_2O$ suspendiert) 45 min. am Rückfluss gekocht. Nach Abkühlung wird abgesaugt, einrotiert und aus Diethylether umkristallisiert. Man erhält 4,3'-Spiro [2,6-dimethyl-3,5-bismethoxy-carbonyl-1,4-dihydropyridin]-[2',3'-dihydro-1'-benzothiophen] mit dem Fp.: 167–168°C.

Ausbeute: 81% der Theorie.

Beispiel 8

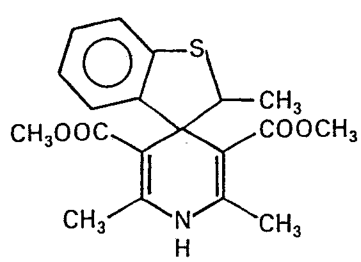

13 mmol 4,3'-Spiro [2,6-dimethyl-3,5-bismethoxy-carbonyl-1,4-dihydropyridin]-[2'-methyl-2',3'-dihydro-1'-benzothiophen-1'-oxid] (Beispiel 4) werden in 150 ml Aceton zusammen mit 50 g Raney-Nickel (in $H_2O$ suspendiert) 30 min. am Rückfluss gekocht, abgesaugt, eingeengt und der Rückstand mit Ether versetzt. Man erhält 4,3'-Spiro [2,6-dimethyl-3,5-bismethoxy-carbonyl-1,4-dihydropyridin]-[2'-methyl-2',3'-dihydro-1'-benzothiophen] mit dem Fp.: 130–131°C.

Ausbeute: 10% der Theorie.

Beispiel 9

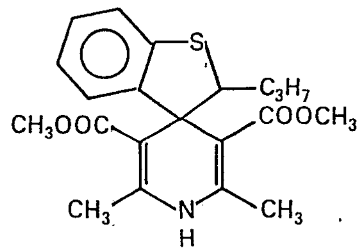

10 mmol 4,3'-Spiro [2,6-dimethyl-3,5-bismethoxy-carbonyl-1,4-dihydropyridin]-[2'-propyl-2',3'-dihydro-1'-benzothiophen-1'-oxid] (Beispiel 5) werden in 200 ml Aceton zusammen mit 40 g Raney-Nickel 1,5 Stunden am Rückfluss gekocht, abgesaugt, eingeengt und der Rückstand an Kieselgel mit Essigester chromatographiert ($R_F$=0,86), Kristalle mit wenig Ether gewaschen. Man erhält 4,3'-Spiro [2,6-dimethyl-3,5-bismethoxy-carbonyl-1,4-dihydropyridin]-[2'-propyl-2',3'-dihydro-1'-benzothiophen] mit dem Fp.: 160–164°C.

Ausbeute: 41% der Theorie.

**Patentansprüche**

1. 4,4-disubstituierte Spiro-1,4-dihydropyridine der allgemeinen Formel (I)

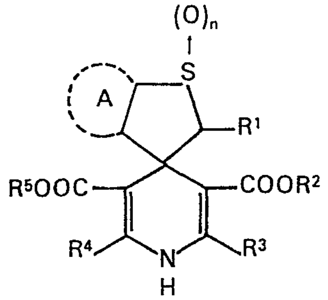

(I)

in welcher

A für Aryl, Furyl, Pyrrolyl, Pyrazolyl, Imidazolyl, Oxazolyl, Isoxazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei der Arylrest sowie die genannten Heterocyclen gegebenenfalls 1, 2 oder 3 gleiche oder verschiedene Substituenten aus der Gruppe Phenyl, Alkyl, Alkenyl, Alkinyl, Alkoxy, Alkylen, Dioxialkylen, Halogen, Trifluormethyl, Trifluormethoxy, Alkylamino, Nitro, Cyano, Azido oder Carbonamido enthalten,

$R^3$ und $R^4$ gleich oder verschieden sind und jeweils für Wasserstoff oder einen geradkettigen oder verzweigten Alkylrest ohne asymmetrisches Kohlenstoffatom, einen Arylrest oder einen Aralkylrest stehen,

$R^2$ und $R^5$ gleich oder verschieden sind und jeweils für einen geradkettigen oder verzweigten aliphatischen oder cycloaliphatischen, gesättigten oder ungesättigten Kohlenwasserstoffrest stehen, der durch ein Sauerstoffatom in der Kette unterbrochen sein kann, und der gegebenenfalls substitutiert ist durch Halogen, Pyridyl, Phenyl oder Phenoxy, wobei die Phenylgruppen ihrerseits substituiert sein können durch Halogen, Cyano, Dialkylamino, Alkoxy, Alkyl, Trifluormethyl oder Nitro,

$R^1$ für Wasserstoff, einen geradkettigen oder verzweigten aliphatischen oder cycloaliphatischen, gesättigten oder ungesättigten Kohlenwasserstoffrest steht, der gegebenenfalls durch ein Sauerstoffatom in der Kette unterbrochen ist und der gegebenenfalls substituiert ist durch Phenyl, Phenoxy, Pyridyl oder Amino, wobei der Phenyl- und der Phenoxyrest ihrerseits gegebenenfalls substituiert sind durch Halogen, Cyano, Dialkylamino, Alkoxy, Alkyl, Trifluormethyl oder Nitro und wobei die Aminogruppe gegebenenfalls substituiert ist durch zwei gleiche oder verschiedene Substituenten aus der Gruppe Alkyl, Alkoxyalkyl, Aryl oder Aralkyl, und

n für 0, 1 oder 2 steht.

2. Verbindungen der allgemeinen Formel (I) gemäss Anspruch 1, in welcher

A für Phenyl, Naphthyl, Furyl, Pyrrolyl oder Pyridyl steht, wobei diese Ringe gegebenenfalls 1, 2 oder 3 gleiche oder verschiedene Substituenten aus der Gruppe Phenyl, Alkyl, Alkenyl, Alkinyl, Alkoxy, Alkylen, Dioxyalkylen, Alkylamino mit jeweils bis zu 4 Kohlenstoffatomen, Halogen, Trifluormethyl, Trifluormethoxy, Nitro, Cyano, Acido oder Carbonamido enthalten,

$R^3$ und $R^4$ gleich oder verschieden sind und jeweils für Wasserstoff oder einen Alkylrest ohne asymmetrisches Kohlenstoffatom mit bis zu 4 Kohlenstoffatomen, einen Phenylrest oder einen Benzylrest stehen,

$R^2$ und $R^5$ gleich oder verschieden sind und jeweils für einen geradkettigen oder verzweigten aliphatischen oder cycloaliphatischen gesättigten oder ungesättigten Kohlenwasserstoffrest mit bis zu 6 Kohlenstoffatomen stehen, der durch ein Sauerstoffatom in der Kette unterbrochen sein kann und der gegebenenfalls substituiert ist durch Halogen, Pyridyl, Phenyl oder Phenoxy, wobei die Phenylgruppen ihrerseits substituiert sein können durch Halogen, Cyano, Nitro oder Trifluormethyl,

$R^1$ für Wasserstoff, einen geradkettigen oder verzweigten aliphatischen oder cycloaliphatischen gesättigten oder ungesättigten Kohlenwasserstoffrest mit bis zu 8 Kohlenstoffatomen steht, der gegebenenfalls in der Kette durch ein Sauerstoffatom unterbrochen ist und der gegebenenfalls substituiert ist durch Phenyl, Phenoxy, Pyridyl oder Amino, wobei der Phenyl- und der Phenoxyrest gegebenenfalls substituiert sind durch Halogen, Cyano, Trifluormethyl, Nitro, Alkyl, Alkoxy oder Dialkylamino mit jeweils 1 bis 4 Kohlenstoffatomen, und wobei die Aminogruppe gegebenenfalls substituiert ist durch zwei gleiche oder verschiedene Substituenten aus der Gruppe Alkyl, Alkoxyalkyl mit jeweils bis zu 4 Kohlenstoffatomen, Phenyl oder Benzyl, und

n für 0, 1 oder 2 steht.

3. Verbindungen der allgemeinen Formel (I) gemäss Anspruch 1, in welcher

A für Phenyl, Furyl, Pyrrolyl oder Pyridyl steht wobei die genannten Ringe gegebenenfalls 1 oder 2 gleiche oder verschiedene Substituenten aus der Gruppe Phenyl, Alkyl, Alkoxy, Alkylamino, mit jeweils 1 bis 4 Kohlenstoffatomen in den Alkyl- und Alkoxyresten, Halogen, Trifluormethyl, Trifluormethoxy, Nitro, Cyano oder Acido enthalten,

$R^3$ und $R^4$ gleich oder verschieden sind und jeweils für Wasserstoff, Alkyl ohne asymmetrisches Kohlenstoffatom mit 1 bis 4 Kohlenstoffatomen, Phenyl oder Benzyl stehen,
$R^2$ und $R^5$ gleich oder verschieden sind und jeweils für einen Alkylrest mit 1 bis 6 Kohlenstoffatomen stehen, der gegebenenfalls durch ein Sauerstoffatom in der Kette unterbrochen ist oder der gegebenenfalls durch Halogen oder Phenyl substituiert ist,
$R^1$ für Wasserstoff, Alkyl oder Alkinyl mit bis zu 6 Kohlenstoffatomen steht, wobei der Alkylrest gegebenenfalls durch ein Sauerstoffatom in der Kette unterbrochen ist und gegebenenfalls substituiert ist durch Phenyl, Phenoxy oder Amino, wobei die Aminogruppe gegebenenfalls substituiert ist durch zwei gleiche oder verschiedene Substituenten aus der Gruppe Alkyl mit 1 bis 4 Kohlenstoffatomen, Phenyl oder Benzyl und
n für 0 oder 1 steht.

4. Verfahren zur Herstellung von Verbindungen der allgemeinen Formel (I) gemäss Anspruch 1, dadurch gekennzeichnet, dass man Pyridine der allgemeinen Formel (II)

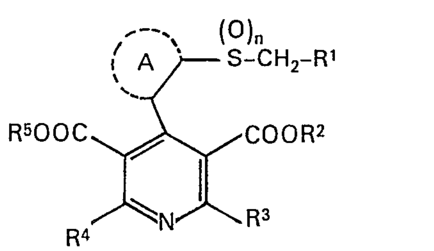

(II)

in welcher
$R^1$, $R^2$, $R^3$, $R^4$, $R^5$, n und A die in Anspruch 1 angegebene Bedeutung haben, bei Temperaturen zwischen −120°C bis +30°C in Gegenwart von mindestens äquivalenten Mengen an Metallhydriden, Metallamiden oder Metallalkoholaten und gegebenenfalls in Gegenwart von inerten organischen Lösungsmitteln zu Spiroverbindungen der allgemeinen Formel (I) cyclisiert.

5. Verfahren gemäss Anspruch 4, dadurch gekennzeichnet, dass man die Cyclisierung in einem Temperaturbereich von −90 bis −40°C durchführt.

6. Verwendung von Verbindungen der allgemeinen Formel (I) gemäss Anspruch 1 zur Herstellung von 4,4-disubstituierten 1,4-Dihydropyridinen der Formel (III)

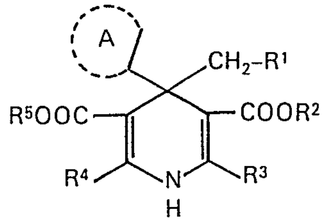

(III)

worin A, $R^1$, $R^2$, $R^3$, $R^4$ und $R^5$ die in Anspruch 1 angegebene Bedeutung haben.

7. Verfahren zur Herstellung von Verbindungen der Formel (I) gemäss Anspruch 1, in welcher n für 0 steht, dadurch gekennzeichnet, dass man die entsprechenden Sulfinylverbindungen der allgemeinen Formel (I) nach an sich bekannten Methoden zu den Benzothiophenverbindungen reduziert.

**Revendications**

1. Spiro-1,4-dihydropyridines disubstituées en 4,4 et répondant à la formule générale (I):

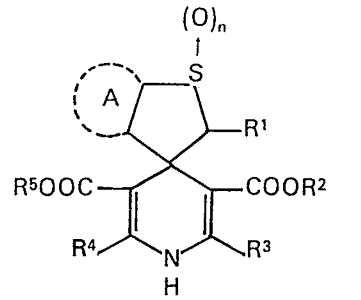

(I)

dans laquelle:
A représente un radical aryle, furyle, pyrrolyle, pyrazolyle, imidazolyle, oxazolyle, isoxazolyle, pyridyle, pyridazinyle, pyrimidyle, pyrazinyle, quinolyle, isoquinolyle, indolyle, benzimidazolyle, quinazolyle ou quinoxalyle, le radical aryle ainsi que les hétérocycles cités comportant éventuellement 1, 2 ou 3 substituants identiques ou différents choisis dans l'ensemble formé par un groupe phényle, alkyle, alcényle, alcynyle, alcoxy, alkylène, dioxyalkylène, halogéno, trifluorométhyle, trifluorométhoxy, alkylamino, nitro, cyano, azido ou carboxamido,
$R^3$ et $R^4$ sont identiques ou différents et représentent chacun un atome d'hydrogène ou un radical alkyle linéaire ou ramifié sans atome de carbone asymétrique, un radical aryle ou un radical aralkyle,
$R^2$ et $R^5$ sont identiques ou différents et représentent chacun un radical d'hydrocarbure aliphatique linéaire ou ramifié ou cycloaliphatique, saturé ou insaturé, dont la chaîne peut être interrompue par un atome d'oxygène, et qui est éventuellement substitué par de l'halogène ou un groupe pyridyle, phényle ou phénoxy, les groupes phényle pouvant pour leur part être substitués par de l'halogène, par un radical cyano, dialkylamino, alcoxy, alkyle, trifluorométhyle ou nitro,
$R^1$ représente un atome d'hydrogène, un radical d'hydrocarbure aliphatique linéaire ou ramifié ou cycloaliphatique, saturé ou insaturé, qui est éventuellement interrompu dans sa chaîne par un atome d'oxygène et qui est éventuellement substitué par un radical phényle, phénoxy, pyridyle ou amino, le radical phényle et le radical phénoxy étant éventuellement substitués pour leur part par de l'halogène ou par un groupe cyano, dialkylamino, alcoxy, alkyle, trifluorométhyle ou nitro et le groupe amino étant éventuellement substitué par deux substituants identiques ou différents choisis dans l'ensemble formé par un groupe alkyle, alcoxyalkyle, aryle ou aralkyle, et
n vaut 0, 1 ou 2.

2. Composés de formule générale (I) selon la revendication 1, dans laquelle:

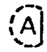 représente un radical phényle, naphtyle, furyle, pyrrolyle ou pyridyle, ces noyaux contenant éventuellement 1, 2 ou 3 substituants identiques ou différents choisis dans l'ensemble formé par un radical phényle, alkyle, alcényle, alcynyle, alcoxy, alkylène, dioxyalkylène, alkylamino ayant chacun jusqu'à 4 atomes de carbone, halogéno, trifluorométhyle, trifluorométhoxy, nitro, cyano, azido ou carboxamido,

$R^3$ et $R^4$ sont identiques ou différents et représentent chacun un atome d'hydrogène ou un radical alkyle sans atome de carbone asymétrique et ayant jusqu'à 4 atomes de carbone, un radical phényle ou un radical benzyle,

$R^2$ et $R^5$ sont identiques ou différents et représentent chacun un radical d'hydrocarbure aliphatique linéaire ou ramifié ou cycloaliphatique, saturé ou insaturé, ayant jusqu'à 6 atomes de carbone, dont la chaîne peut être interrompue par un atome d'oxygène, et qui est éventuellement substitué par de l'halogène ou par un radical pyridyle, phényle ou phénoxy, les groupes phényle pouvant être substitués pour leur part par de l'halogène ou par un groupe cyano, nitro ou trifluorométhyle,

$R^1$ représente un atome d'hydrogène, un radical d'hydrocarbure aliphatique linéaire ou ramifié ou cycloaliphatique, saturé ou insaturé, ayant jusqu'à 8 atomes de carbone, dont la chaîne est éventuellement interrompue par un atome d'oxygène, et qui est éventuellement substitué par un radical phényle, phénoxy, pyridyle ou amino, le radical phényle et le radical phénoxy étant éventuellement substitués par de l'halogène ou par un groupe cyano, trifluorométhyle, nitro, alkyle, alcoxy ou dialkylamino ayant chacun 1 à 4 atomes de carbone, et le groupe amino étant éventuellement substitué par deux substituants identiques ou différents choisis dans l'ensemble formé par un groupe alkyle ou alkoxyalkyle ayant chacun jusqu'à 4 atomes de carbone, et

n vaut 0, 1 ou 2.

3. Composés de formule générale (I) selon la revendication 1, dans laquelle:

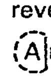 représente un radical phényle, furyle, pyrrolyle ou pyridyle, les noyaux cités contenant éventuellement un ou deux substituants identiques ou différents choisis dans l'ensemble formé par un radical phényle, alkyle, alcoxy, alkylamino ayant chaque fois 1 à 4 atomes de carbone dans les radicaux alkyle et alcoxy, halogéno, trifluorométhyle, trifluorométhoxy, nitro, cyano ou azido,

$R^3$ et $R^4$ sont identiques ou différents et représentent chacun un atome d'hydrogène, un radical alkyle sans atome de carbone asymétrique et ayant 1 à 4 atomes de carbone, un radical phényle ou benzyle,

$R^2$ et $R^5$ sont identiques ou différents et représentent chacun un radical alkyle ayant 1 à 6 atomes de carbone, dont la chaîne est éventuellement interrompue par un atome d'oxygène, et qui est éventuellement substitué par de l'halogène ou par un radical phényle.

$R^1$ représente un atome d'hydrogène ou un radical alkyle ou alcynyle ayant jusqu'à 6 atomes de carbone, le radical alkyle pouvant éventuellement être interrompu dans sa chaîne par un atome d'oxygène et être éventuellement substitué par un radical phényle, phénoxy ou amino, le groupe amino étant éventuellement substitué par deux substituants identiques ou différents choisis parmi un groupe alkyle ayant 1 à 4 atomes de carbone, un radical phényle ou benzyle, et

n vaut 0 ou 1.

4. Procédé de préparation de composés de formule générale (I) selon la revendication 1, caractérisé en ce qu'on cyclise des pyridines de formule générale (II):

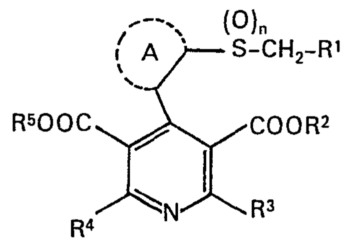

(II)

dans laquelle:

($R^1$, $R^2$, $R^3$, $R^4$, $R^5$,n et A ont le sens indiqué à la revendication 1) à des températures comprises entre −120°C et +30°C en présence de quantités au moins équivalentes d'hydrures de métaux, d'amidures de métaux ou d'alcoolates de métaux et éventuellement en présence de solvants organiques inertes pour obtenir des composés du type spiro répondant à la formule générale (I).

5. Procédé selon la revendication 4, caractérisé en ce qu'on effectue la cyclisation dans une gamme de températures comprises entre −90°C et −40°C.

6. Utilisation de composés de formule générale (I) selon la revendication 1 pour la préparation de 1,4-dihydropyridines disubstituées en 4,4 et répondant à la formule (III):

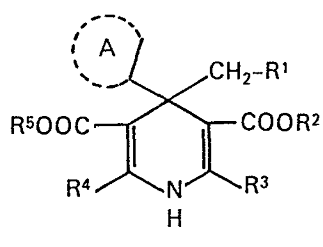

(III)

dans laquelle:

A, $R^1$, $R^2$, $R^3$, $R^4$ et $R^5$ ont le sens indiqué à la revendication 1.

7. Procédé de préparation de composés de formule (I) selon la revendication 1, dans laquelle n est nul, procédé caractérisé en ce qu'on réduit selon des méthodes connues en elles-mêmes les composés sulfinyliques correspondants de formule générale (I) pour obtenir les composés dérivés du benzothiophène.

## Claims

1. 4,4-Disubstituted spiro-1,4-dihydropyridines of the general formula (I)

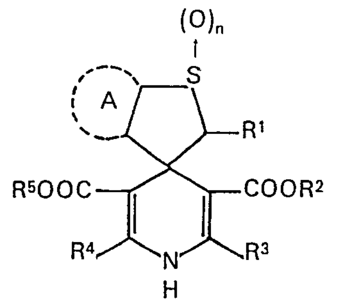

(I)

in which
A represents aryl, furyl, pyrrolyl, pyrazolyl, imidazolyl, oxazolyl, isoxazolyl, pyridyl, pyridazinyl, pyrimidyl, pyrazinyl, quinolyl, isoquinolyl, indolyl, benzimidazolyl, quinazolyl or quinoxalyl, the aryl radical and the heterocyclic radicals mentioned optionally containing 1, 2 or 3 identical or different substituents from the group comprising phenyl, alkyl, alkenyl, alkinyl, alkoxy, alkylene, dioxyalkylene, halogen, trifluoromethyl, trifluoromethoxy, alkylamino, nitro, cyano, azido or carboxamido,
$R^3$ and $R^4$ are identical or different and each represent hydrogen or a straight-chain or branched alkyl radical having no asymmetric carbon atom, an aryl radical or an aralkyl radical,
$R^2$ and $R^5$ are identical or different and each represent a straight-chain or branched, aliphatic or cycloaliphatic, saturated or unsaturated hydrocarbon radical, which can be interrupted in the chain by one oxygen atom and is optionally substituted by halogen, pyridyl, phenyl or phenoxy, it being possible for the phenyl groups in turn to be substituted by halogen, cyano, dialkylamino, alkoxy, alkyl, trifluoromethyl or nitro,
$R^1$ represents hydrogen or a straight-chain or branched, aliphatic or cycloaliphatic, saturated or unsaturated hydrocarbon radical which is optionally interrupted in the chain by one oxygen atom and is optionally substituted by phenyl, phenoxy, pyridyl or amino, the phenyl and the phenoxy radical being in turn optionally substituted by halogen, cyano, dialkylamino, alkoxy, alkyl, trifluoromethyl or nitro, and the amino group optionally being substituted by two identical or different substituents from the group comprising alkyl, alkoxyalkyl, aryl and aralkyl, and
n represents 0, 1 or 2.

2. Compounds of the general formula (I) according to Claim 1, in which

A represents phenyl, naphthyl, furyl, pyrrolyl or pyridyl, these rings optionally containing 1, 2 or 3 identical or different substituents from the group comprising phenyl, alkyl, alkenyl, alkinyl, alkoxy, alkylene, dioxyalkylene and alkylamino with in each case up to 4 carbon atoms, halogen, trifluoromethyl, trifluoromethoxy, nitro, cyano, acido and carboxamido,
$R^3$ and $R^4$ are identical or different and each represent hydrogen or an alkyl radical with up to 4 carbon atoms having no asymmetric carbon atom, a phenyl radical or a benzyl radical,
$R^2$ and $R^5$ are identical or different and each represent a straight-chain or branched, aliphatic or cycloaliphatic, saturated or unsaturated hydrocarbon radical which has up to 6 carbon atoms, and which can be interrupted in the chain by one oxygen atom and is optionally substituted by halogen, pyridyl, phenyl or phenoxy, it being possible for the phenyl groups in turn to be substituted by halogen, cyano, nitro or trifluoromethyl,
$R^1$ represents hydrogen, a straight-chain or branched, aliphatic or cycloaliphatic, saturated or unsaturated hydrocarbon radical which has up to 8 carbon atoms, and which is optionally interrupted in the chain by one oxygen atom and is optionally substituted by phenyl, phenoxy, pyridyl or amino, the phenyl and phenoxy radical optionally being substituted by halogen, cyano, trifluoromethyl, nitro, alkyl, alkoxy or dialkylamino with in each case 1 to 4 carbon atoms, and the amino group optionally being substituted by two identical or different substituents from the group comprising alkyl and alkoxyalkyl with in each case up to 4 carbon atoms, and
n represents 0, 1 or 2.

3. Compounds of the general formula (I) according to Claim 1, in which

A represents phenyl, furyl, pyrrolyl or pyridyl, the rings mentioned optionally containing 1 or 2 identical or different substituents from the group comprising phenyl, alkyl, alkoxy and alkylamino with in each case 1 to 4 carbon atoms in the alkyl and alkoxy radicals, halogen, trifluoromethyl, trifluoromethoxy, nitro, cyano or acido,
$R^3$ and $R^4$ are identical or different and each represent hydrogen, alkyl with 1 to 4 carbon atoms having no asymmetric carbon atom, phenyl or benzyl,
$R^2$ and $R^5$ are identical or different and each represent an alkyl radical which has 1 to 6 carbon atoms, and which is optionally interrupted in the chain by one oxygen atom or is optionally substituted by halogen or phenyl,
$R^1$ represents hydrogen, alkyl or alkinyl with up to 6 carbon atoms, the alkyl radical optionally being interrupted in the chain by one oxygen atom and optionally being substituted by phenyl, phenoxy or amino, the amino group optionally being substituted by two identical or different substituents from the group comprising alkyl with 1 to 4 carbon atoms, phenyl and benzyl, and
n represents 0 or 1.

4. Process for the preparation of compounds of the general formula (I) according to Claim 1, characterised in that pyridines of the general formula (II)

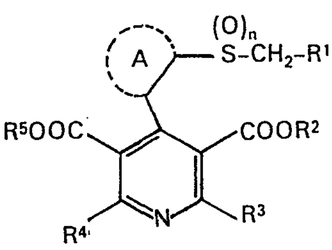

(II)

in which

$R^1$, $R^2$, $R^3$, $R^4$, $R^5$, n and A have the meaning indicated in Claim 1,

are cyclised at temperatures between −120°C and +30°C in the presence of at least equivalent amounts of metal hydrides, metal amides or metal alcoholates, and if appropriate in the presence of inert organic solvents, to give spiro compounds of the general formula (I).

5. Process according to Claim 4, characterised in that the cyclisation is carried out in a temperature range from −90 to −40°C.

6. Use of compounds of the general formula (I) according to Claim 1 for the preparation of 4,4-disubstituted 1,4-dihydropyridines of the formula (III)

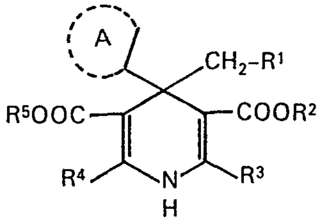

(III)

wherein

A, $R^1$, $R^2$, $R^3$, $R^4$ and $R^5$ have the meaning indicated in Claim 1.

7. Process for the preparation of compounds of the formula (I) according to Claim 1 in which n represents 0, characterised in that the corresponding sulphinyl compounds of the general formula (I) are reduced to the benzothiophene compounds by methods which are in themselves known.